(19) Europäisches Patentamt
European Patent Office
Office européen des brevets

(11) EP 0 994 887 B1

(12) EUROPEAN PATENT SPECIFICATION

(45) Date of publication and mention of the grant of the patent:
**27.11.2002 Bulletin 2002/48**

(21) Application number: **98932361.3**

(22) Date of filing: **03.07.1998**

(51) Int Cl.[7]: **C07H 1/00**

(86) International application number:
**PCT/GB98/01962**

(87) International publication number:
**WO 99/001463 (14.01.1999 Gazette 1999/02)**

(54) **MODIFIED GLYCOSIDES, COMPOSITIONS COMPRISED THEREOF AND METHODS OF USE THEREOF**

MODIFIZIERE GLYKOSIDE, MODIFIZIERTE GLYCKOSIDE ENTHALTENDE ZUSAMMENSETZUNGEN UND VERFAHREN ZU IHRER HERSTELLUNG

GLYCOSIDES MODIFIES, COMPOSITIONS RENFERMANT CES GLYCOSIDES ET PROCEDES D'UTILISATION CONNEXES

(84) Designated Contracting States:
**AT BE CH CY DE DK ES FI FR GB GR IE IT LI LU MC NL PT SE**

(30) Priority: **03.07.1997 US 51727 P**

(43) Date of publication of application:
**26.04.2000 Bulletin 2000/17**

(73) Proprietor: **Elan Drug Delivery Limited**
**Nottingham, NG11 6JS (GB)**

(72) Inventor: **COLACO, Camilo**
**Trumpington, Cambridgeshire CB2 2JN (GB)**

(74) Representative: **Perry, Robert Edward**
**GILL JENNINGS & EVERY**
**Broadgate House**
**7 Eldon Street**
**London EC2M 7LH (GB)**

(56) References cited:
**WO-A-96/03978**

- **BEBAULT, GWENDOLYN M. ET AL: "Synthesis of 4-O-.beta.-D-mannopyranosyl-L-rhamnopyranose" 1974 , CARBOHYDR. RES. (1974), 37(2), 309-19 CODEN: CRBRAT XP002085499 See page 318 compound 18. see page 312, paragraph 1**
- **NANASI, P. ET AL: "Synthesis of O-.alpha.-D-glucopyranosyl-(1.fwdarw. 4)-O-.beta.-D-glucopyranosyl-(1.fwdarw. 6)-D-glucose" 1976 , ACTA CHIM. ACAD. SCI. HUNG. (1976), 88(2), 154-8 CODEN: ACASA2 XP002085500 See Structure 8 page 158.**
- **STANLEY PEAT ET AL.: "The structure of Laminarin. Part III. Synthesis of structural Oligosaccharides." J. CHEM. SOC. , - 1960 pages 175-178, XP002085498**
- **COLOMBO, DIEGO ET AL: "Optically pure 1-O- and 3-O-.beta.-D-glucosyl- and galactosyl-sn-glycerols through lipase-catalyzed transformations" 1995 , TETRAHEDRON LETT. (1995), 36(27), 4865-8 CODEN: TELEAY;ISSN: 0040-4039 XP004027788 See structures 1-8 on page 4866.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 994 887 B1

Printed by Jouve, 75001 PARIS (FR)

## Description

### TECHNICAL FIELD

This invention relates to compositions comprising modified glycosides of sugar alcohols and a substance to be released from the composition and methods for their use. In the field of pharmaceutical drug delivery, the modified glycosides of sugar alcohols can be used to form solid delivery systems useful for the dissolution, encapsulation, storage and delivery of a variety of therapeutic molecules. In the manufacturing fields, the optically clear nature of the compositions renders them suitable for use as a vehicle for coloring or coating a wide variety of materials such as plastics, metals and glasses.

### BACKGROUND ART

Solid delivery systems are useful in a wide variety of applications such as controlled release of labile molecules, particularly bioactive materials such as organic pharmaceutical compounds, enzymes, vaccines and biological control agents such as pesticides and pheromones.

Drugs and other active agents are frequently administered orally by means of solid dosage forms, such as tablets and capsules. Other oral solid dosage forms include lozenges and other hard candies. Solid dosage forms can also be implanted subcutaneously for drug delivery. Additionally, solid dosage forms are delivered intravenously, or by inhalation to-the pulmonary system.

Solid dose delivery of bioactive materials to biological tissues such as mucosal, dermal, ocular, subcutaneous, intradermal and pulmonary offers several advantages over methods such as hypodermic injection and transdermal administration via so-called "patches". Using injection, there is a risk of infection using conventional needles and syringes. Dosing using multidose vials is sometimes variable, and physical discomfort often attends hypodermic injection. Devices used for administering drugs transdermally usually comprise laminated composites with a reservoir layer of drug with the composite being adhered to the skin, i.e., transdermal patches, such as described in U.S. Patent No. 4,906,463. Many drugs are not suitable for transdermal delivery, nor have transdermal drug release rates for those capable of such delivery been perfected. Additionally, transdermal patches often cause topical reactions, in many instances precluding their long-term use.

Subdermal implantable therapeutic systems have been formulated for slow release of certain pharmaceutical agents for extended periods of time such as months or years. A well-known example is the Norplant® for delivery of steroid hormones. In membrane permeation-type controlled drug delivery, the drug is encapsulated within a compartment enclosed by a rate-limiting polymeric membrane. The drug reservoir can contain either drug particles or a dispersion (or solution) of solid drug in a liquid or a solid type dispersing medium. The polymeric membrane can be fabricated from a homogeneous or a heterogeneous nonporous polymeric material or a microporous or semipermeable membrane. The encapsulation of the drug reservoir inside the polymeric membrane can be accomplished by molding, encapsulation, microencapsulation, or other techniques.

The implants release drugs by dissolution of the drug in the inner core and slow release across the outer matrix. The drug release from this type of implantable therapeutic system is dependent on drug dissolution rate in the polymeric membrane, often causing a biphasic release rate. The inner core substantially dissolves, however, generally, the outer matrix does not dissolve.

Implants are placed subcutaneously by making an incision in the skin and forcing the implants between the skin and the muscle. At the end of their use, if not dissolved, these implants must be surgically removed. United States Patent No. 4,244,949 describes an implant which has an outer matrix of an inert plastic such as polytetrafluoroethylene resin. Examples of this type of implantable therapeutic system are Progestasert IUD and Ocusert system.

Other implantable therapeutic systems involve matrix diffusion-type controlled drug delivery. The drug reservoir is formed by the homogeneous dispersion of drug particles throughout a lipophilic or hydrophilic polymer matrix. The dispersion of drug particles in the polymer matrix is accomplished by blending the drug with a viscous liquid polymer or a semisolid polymer at room temperature, followed by cross-linking of the polymer, or by mixing the drug particles with a melted polymer at an elevated temperature. The drug reservoir can also be fabricated by dissolving the drug particles and/or the polymer in an organic solvent followed by mixing and evaporation of the solvent in a mold at an elevated temperature or under vacuum. The rate of drug release from this type of delivery device is generally not constant. Examples of this type of implantable therapeutic system are the contraceptive vaginal ring and Compudose implant.

A variety of formulations have been provided for administration in aerosolized form to mucosal surfaces, particularly "by-inhalation" (naso-pharyngeal and pulmonary). Compositions for by-inhalation pharmaceutical administration generally comprise a liquid formulation of the pharmaceutical agent and a device for delivering the liquid in aerosolized form. United States Patent No. 5,011,678 describes suitable compositions containing a pharmaceutically

active substance, a biocompatible amphiphilic steroid and a biocompatible (hydro/fluoro) carbon propellant. United States Patent No. 5,006,343 describes suitable compositions containing liposomes, pharmaceutically active substances and an amount of alveolar surfactant protein effective to enhance transport of the liposomes across a pulmonary surface. United States Patent No. 5,608,647 describes methods for administering controlled amounts of aerosol medication from a valved canister.

One drawback to the use of aerosolized formulations is that maintenance of pharmaceutical agents in aqueous suspensions or solutions can lead to aggregation and loss of activity and bioavailability. The loss of activity can be partially prevented by refrigeration; however, this limits the utility of these formulations. The use of powdered formulations overcomes many of these drawbacks. The requisite particle size of such powders is 0.5-5 microns in order to attain deep alveolar deposition in pulmonary delivery. Unfortunately, powders of such particle size tend to absorb water and clump, thus diminishing deposition of the powder in the deep alveolar spaces. WO 96/03978 described powders suitable for use in by-inhalation delivery. The powders are of uniform particle size and can be produced with varying degrees of hydrophobicity to reduce clumping and increase drug release in the surfactant environment of the lung.

Solid dose delivery vehicles for ballistic, transdermal administration have also been developed. For example, in U.S. Patent No. 3,948,263, a ballistic animal implant comprised of an exterior polymeric shell encasing a bioactive material is described for veterinary uses. Similarly, in U.S. Patent No. 4,326,524, a solid dose ballistic projectile comprising bioactive material and inert binder without an exterior casing is disclosed. Delivery is by compressed gas or explosion. Ballistic delivery at the cellular level has also been successful. Klein (1987) Nature 327:70-73. There are few existing formulations suitable for ballistic delivery. Powder formulations of pharmaceuticals generally used are unsuitable for ballistic administration, because they vary in size, shape and density. The particles described in WO 96/03978 are useful for ballistic delivery due to their discrete size.

For drug delivery, it would be advantageous to provide solid drug delivery systems of defined size, shape, density and dissolution rate. It would be advantageous to provide solid drug delivery systems which are capable of sustained, controlled release of the drug. It would be further advantageous to provide solid dose delivery systems which could be formulated using simple and economical methods.

WO 93/10758 describes a carbohydrate glass matrix for the sustained release of a therapeutic agent, which includes a carbohydrate, a therapeutic agent, an agent which inhibits recrystallization of the matrix, and a water insoluble wax which modifies release of the therapeutic agent from the matrix. WO 96/03978 describes solid dose delivery systems shaped for penetrating the epidermis, which include a vitreous vehicle loaded with a guest substance, and which are capable of releasing the guest substance at a controlled rate. WO 96/03978 describes amorphous glassy matrices for formation of a number of controlled release delivery systems.

The use of amorphous matrices for various material science applications such as semiconductors, non-crystalline films, glass ceramics and glassy composites has also been described. ("Glasses and Glass Ceramics from Gels", Ed. S. Sakka. 1987 North-Holland, Amsterdam).

## DISCLOSURE OF THE INVENTION

Compositions comprising modified glycosides and a substance to be released from the composition are provided and methods of use thereof. The modified glycosides include lactitol nonaacetate, palatinit nonaacetate, glucopyranosyl sorbitol nonaacetate, glucopyranosyl mannitol nonaacetate and mixtures thereof. The modified glycosides can be formed by modification of polyol glycosides such as lactitol (4-O-β-D-galactopyranosyl-D-glucitol), palatinit [a mixture of GPS (α-D-glucopyranosyl-1→6-sorbitol) and GPM (α-D-glucopyranosyl-1→6-mannitol)], the individual glycoside components thereof, GPS and GPM, maltitol (4-O-β-D-glucopyranosyl-D-glucitol), hydrogenated maltooligosaccharides (such as maltotritol, maltotetraitol, maltopentaitol, maltohexaitol, maltooctaitol, maltononaitol and maltodecaitol) and hydrogenated isomaltooligosaccharides. The modified glycosides may be, for example, ester or ether derivatives of glycosides, or mixed ester or ether derivatives of glycosides. The modified glycosides can include saccharide and oligosaccharide subunits, such as furanose or pyranose saccharide subunits, or mixtures thereof.

Exemplary structures of modified glycosides are shown below:

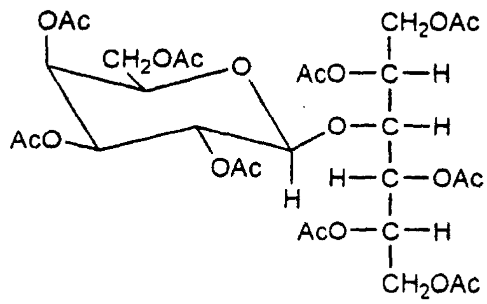

lactitol nonaacetate (4-O-β-D-galactopyranosyl-D-glucitol nonaacetate)

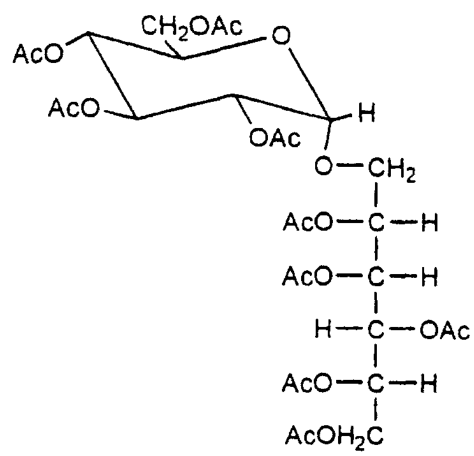

GPS nonaacetate (α-D-glucopyranosyl-l →6-sorbitol nonaacetate)

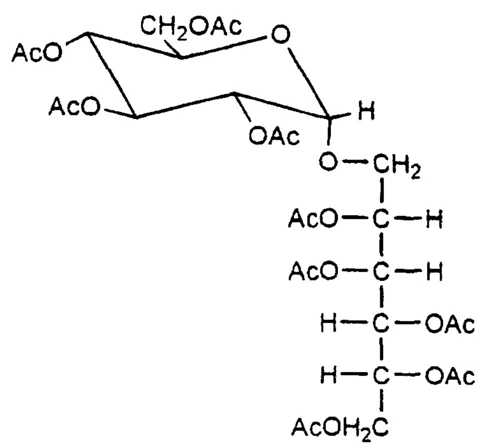

GPM nonaacetate (α-D-glucopyranosyl-l →6-mannitol nonaacetate)

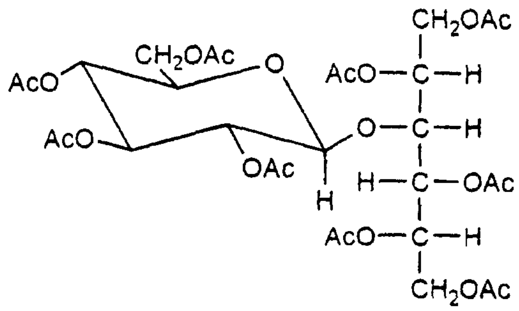

maltitol nonaacetate (4-O-β-D-glucopyranosyl-D-glucitol nonaacetate)

In one embodiment, the modified glycosides are represented by Formula I or Formula II shown below.

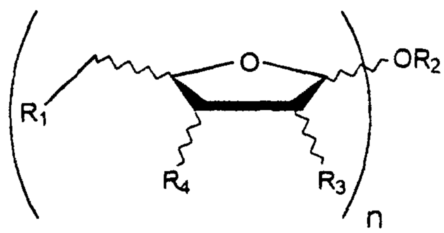

I

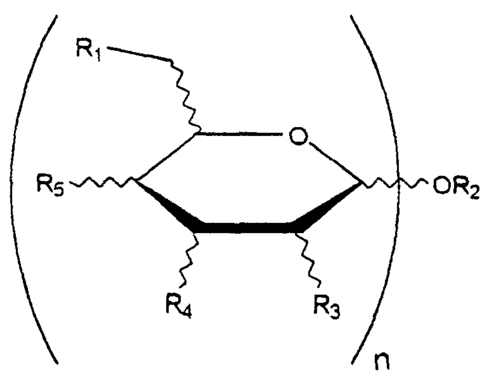

II

wherein $R_1$, $R_3$, $R_4$ and $R_5$ are independently OH, $NH_2$, $NHR_6$, $N(R_6)_2$, $OR_6$ or $O(C{=}O)R_6$, wherein $R_6$ is alkyl, preferably a straight chain or branched, saturated or unsaturated, C1-C25 hydrocarbon, such as a C1-C15 hydrocarbon, or in one preferred embodiment, a C1-C8 hydrocarbon, for example, methyl or isobutyl;

wherein $OR_2$ is a monosaccharide polyalcohol, preferably a reduced monosaccharide 5 or 6 carbon polyalcohol, such as ribitol, xylitol, mannitol or glucitol; and

wherein n is 1-6, where each subunit, n, may include the same or different substituents, $R_1$, $R_3$, $R_4$ and $R_5$ and wherein the subunits are linked in a linear or branched chain via a C N or O linkage at the positions $R_1$, $R_3$, $R_4$ or $R_5$.

Modified glycosides within the scope of the invention include modified glycosides of sugar alcohols, also referred to herein as hydrogenated oligosaccharides. The modified glycosides are in one embodiment derivatives of hydrogenated maltooligosaccharides or derivatives of hydrogenated isomaltooligosaccharides. As used herein, the term "hydrogenated oligosaccharide" refers to an oligosaccharide including preferably about 2 to 7 saccharide units, wherein a terminal saccharide subunit is reduced and is in the form of a polyalcohol. As used herein, the term "hydrogenated maltooligosaccharide" refers to a branched or straight chain oligosaccharide including about 2 to 7 glucose units, linked by glycoside linkage, wherein a terminal glucose subunit is reduced and is in the form of the polyalcohol,

glucitol. As used herein, the term "hydrogenated isomaltooligosaccharide" refers to a branched oligosaccharide including about 2 to 7 glucose units, linked by glycoside linkage, wherein a terminal fructose subunit is reduced and is in the form of the polyalcohols sorbitol or mannitol.

The modified glycosides in one embodiment are glycosides which are derivatized to render them hydrophobic, for example, by the esterification of at least a portion of free hydroxyl groups on the glycoside with fatty acid acyl groups. The modified glycoside in one embodiment is a hydrophobic ester or mixed ester derivative of a glycoside of a sugar alcohol. In one preferred embodiment, the modified glycoside is a hydrophobic derivative of a hydrogenated maltooligosaccharide, which is rendered hydrophobic by derivatization of the free hydroxyl groups, for example, to form fatty acid acyl esters or long hydrocarbon chain ethers. In one embodiment, the modified glycosides are represented by compounds of Formula III below:

$$(Y)_n\text{-}X$$

III

where Y represents a saccharide subunit, or derivative thereof, and n is 1-6, wherein each of the n saccharide subunits are linked in a linear or branched chain by glycosidic linkages; and where X is a 5 or 6 carbon monosaccharide polyalcohol, such as ribitol, xylitol, mannitol or glucitol. For example, $(Y)_n$ may be a branched or straight chain oligosaccharide including glucose subunits which are linked by an α-or β-glucosidic linkage, such as a 1→6 or 1→4 linkage, and X can be a polyalcohol linked via a glycosidic bond to an anomeric carbon on one of the glucose subunits. In the compounds of Formula III, all or a portion of the free hydroxyl groups in the saccharide subunits and the polyalcohol are derivatized in the form of esters, ethers, mixed esters or mixed ethers. For example, the free hydroxyl groups may be reacted with the appropriate reagent to form acyl esters, isobutyl esters, or esters of C1-C25 saturated or unsaturated branched or straight chain fatty acids, or mixtures thereof. The modification of the hydroxyl groups with the ester or ether functionalities thus can render the compound hydrophobic. An exemplary compound is shown below:

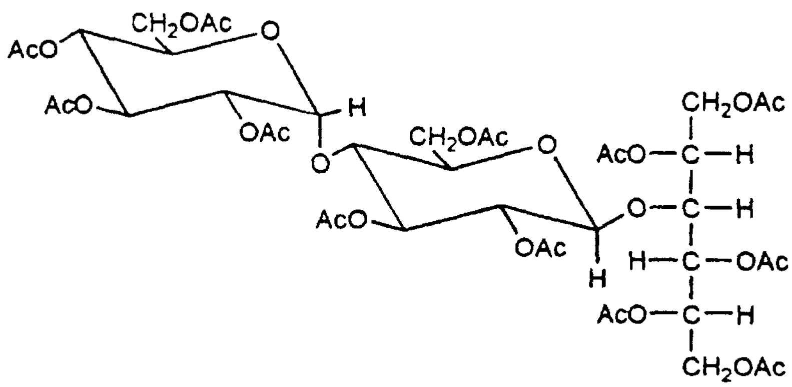

4-O-(α-D-glucopyranosyl)-4-O-(β-D-glucopyranosyl)-D-glucitol dodecaacetate

Compositions comprising the modified glycosides, and other components such as bioactives, carbohydrates, binders, and any other constituent suitable for use in drug delivery are also encompassed by the invention. A wide variety of compositions can be incorporated into the solid delivery systems including diagnostic, therapeutic, prophylactic and other biologically active agents.

Solid delivery systems are provided, which comprise a modified glycoside having incorporated therein a substance capable of being released from the solid delivery system. The release rate of the substance can be modulated by the addition of different glass formers with known release rates. In a preferred embodiment, the solid delivery system comprises the modified glycoside in the form of a vitreous glass matrix having the substance incorporated therein. In one preferred embodiment, the modified glycoside in the solid delivery systems is an acetylated glycoside. Preferred modified glycosides are lactitol nonaacetate, palatinit nonaacetate, glucopyranosyl sorbitol nonaacetate, glucopyranosyl mannitol nonaacetate or maltitol nonaacetate.

The invention further encompasses compositions comprising a modified glycoside and a second physiologically acceptable glass material, such as a carboxylate, nitrate, sulfate, bisulfate, or combinations thereof. The delivery systems can further incorporate any other carbohydrate and/or hydrophobic carbohydrate derivative (HDC), such as trehalose octaacetate.

The solid delivery systems can be in any of a variety of forms including a lozenge, tablet, disc, film, suppository, needle, microneedle, microfiber, particle, microparticle, sphere, microsphere, powder, or an implantable device.

The invention further encompasses methods of making the solid delivery systems. In one embodiment, the method comprises forming a modified glycoside capable of forming a vitreous glass; processing the modified glycoside and a substance to be released therefrom, and forming a vitreous glass matrix having the substance incorporated therein.

The processing step can be implemented by melting the modified glycoside and incorporating the substance in the melt, at a melt temperature sufficient to fluidize the modified glycoside, and insufficient to substantially inactivate the substance, and then quenching the melt. The melt can be processed into a variety of forms. The processing step can be further implemented by dissolving or suspending the modified glycoside and the substance in a solvent effective in dissolving at least one of the modified glycosides and the substance, and evaporating the solvent.

Methods of making the modified glycosides are also provided. The modified glycoside can be provided in one embodiment by acetylating free hydroxyl groups on a glycoside, to form the modified glycoside. In one embodiment, lactitol, palatinit, glycopyranosyl sorbitol or glycopyranosyl mannitol are acetylated to form the modified glycosides, lactitol nonaacetate, palatinit nonaacetate, glucopyranosyl sorbitol nonaacetate, and glucopyranosyl mannitol nonaacetate, respectively.

The invention further encompasses glass matrices comprising the modified glycosides. The qualities of the glass matrices can be modified by choice of modified carbohydrate, and other incorporated materials, to have a desired rate of release of the incorporated substance. Other materials can be incorporated into the glass matrix during processing to modify the properties of the final composition, including physiologically acceptable glasses such as carboxylate. nitrate, sulfate, bisulfate, and combinations thereof.

The invention also encompasses methods of delivering bioactive materials by providing the solid dose delivery systems described above and administering the system to a biological tissue. Administration can be by any suitable means including mucosal, oral, topical, subcutaneous, intraperitoneal, intradermal, intramuscular, intravenous and by-inhalation.

The delivery systems are uniquely suited to delivery of hydrophobic substances such as pesticides, pheromones, steroid hormones, peptides, peptide mimetics, antibiotics and other organic pharmaceuticals such as synthetic corticosteroids, bronchodilators, immunomodulators and immunosuppressants. The invention encompasses these delivery systems. The delivery systems are also suitable for delivery of a wide variety of non-medical substances, such as compounds used in agricultural applications, including pesticides, or enzymes or other substances added to laundry detergents. The invention also encompasses delivery systems for non-medical use.

The invention also encompasses optically clear compositions comprising the modified glycosides encapsulating photoactive molecules such as dyes. The modified glycosides have been found to have excellent solvent properties for the dissolution of a number of poorly water soluble intense dyes. These compositions are thus particularly suitable for use as coatings or layers for various applications. Furthermore the significant intensification of colour obtained when these dyes are in true solution enables compositions to be formed with minimal quantities of expensive photoactive materials. These compositions are particularly useful as they remain clear under conditions of ambient temperature and humidity due to their extremely slow rates of devitrification.

The invention further encompasses methods of use of the optically clear glass matrices. The glass matrices are suitable for use in providing dyes in forming colored plastics such as in the form of colored pellets for mixing, or by co-extrusion. The glass matrices are also suitable for use in coating a wide variety of matrices such as glasses, plastics and other solid surfaces. Coating is accomplished by any method known in the art, particularly by sputtering.

## BEST MODE FOR CARRYING OUT THE INVENTION

Modified glycosides of sugar alcohols are provided, which are particularly useful in forming vitreous glass matrices. The modifications include ester and ether derivatives in either single or mixed compositions. A wide variety of substances can be incorporated into the glass matrices, including diagnostic, therapeutic, prophylactic, antimicrobial, insecticidal, environmental, and other bioactive agents and dyes, photochromes and other colored substances. In the medical field, the glass matrices are useful as biodegradable solid materials for controlled delivery and release of the incorporated substance. In the field of materials manufacture, the glass matrices are also useful as coatings that do not release solid materials incorporated therein.

The modified glycosides are formed in one embodiment by the esterification of the free hydroxyl groups on a glycoside. Preferred modified glycosides within the scope of the invention include, but are not limited to, lactitol nonaacetate, palatinit nonaacetate, glucopyranosyl sorbitol nonaacetate, and glucopyranosyl mannitol nonaacetate. The modified glycosides are useful in forming vitreous glasses which can be processed into different solid forms, including tablets, powders, lozenges, implants and microspheres.

The use of gel-sol techniques for the formation of glassy matrices has enabled applications such as monoliths,

fibres coating films etc. "Glasses and Glass Ceramics From Gels," Ed., S. Sakka. (1987) North-Holland, Amsterdam. These applications can now be extended using techniques of formation of glassy matrices by solvent evaporation, and/or from the melt, if organic glass formers are used. Particular advantages of the group of novel organic glass forming modified glycosides described herein is their low cost, biodegradability, ease of synthesis and good solvent properties for various actives including organic molecules such as bioactives and optical actives (e.g. dyes and photochromes) and even inorganic compounds such as mixed transition metal oxides and metal alkoxides.

*Formation of Modified Glycosides*

The modified glycosides are formed in one embodiment by the esterification of the free hydroxyl groups on a glycoside. For example, all of the free hydroxyl groups can be esterified with acetic acid or propionic acid, or mixtures thereof. Alternatively, partial or mixed esters can be formed.

Methods for esterifying the glycosides are available in the art. For example, the glycosides can be treated with sodium acetate in acetic anhydride to form the acetylated polyol. Additionally, partial or mixed esters can be formed by manipulation of the reaction conditions and reagent amounts Such partial and/or mixed esters are also encompassed by the invention.

A variety of modified glycosides are within the scope of the invention. For example, polyol glycosides of sugar alcohols may be esterified with acetyl groups. In a preferred embodiment, the polyols are lactitol (4-O-β-D-galactopyranosyl-D-glucitol), palatinit [a mixture of GPS (α-D-glucopyranosyl-1→6-sorbitol) and GPM (α-D-glucopyranosyl-1→6-mannitol)], and the individual glycoside components thereof, GPS (also referred to herein as glucopyranosyl sorbitol) and GPM (also referred to herein as glucopyranosyl mannitol). Additionally, the polyol can be maltitol (4-O-β-D-glucopyranosyl-D-glucitol), or hydrogenated maltooligosaccharides and isomaltooligosaccharides.

In one embodiment, the glycoside is esterified and treated with sodium acetate and acetic anhydride. Examples of this include, but are not limited to, esterification of lactitol, palatinit, GPS or GPM treated with sodium acetate and acetic anhydride, to form respectively, lactitol nonaacetate, palatinit nonaacetate, glucopyranosyl sorbitol nonaacetate, and glucopyranosyl mannitol nonaacetate.

The reaction product can be structurally characterized by nuclear magnetic resonance spectroscopy (NMR) and its material science properties characterized by differential scanning calorimetry (DSC). The characteristic melting points and Tgs (glass transition temperatures) for the modified glycosides can also be determined by DSC and other methods known in the art.

The Tgs of the compositions encompassed herein are low, typically less than about 200°C and, surprisingly, are not predictable from the melt temperatures. In general, the tendency of the glass matrices described herein to crystallize, from the melt or with reducing solvent, is low. Glasses formed using the modified glycosides preferably have melt temperatures suitable for the incorporation of substances such as biologically active compounds, without thermal degradation, and have Tgs above ambient temperatures.

Both devitrification and the fluidity of the melt at temperatures close to Tg, can be controlled by modifiers such as other derivative sugars and certain organic compounds. Suitable derivative sugars and organic compounds are described for instance, in PCT GB95/01861.

As used herein, ambient temperatures are those of the surrounding environment of any given environment. Typically, ambient temperatures are "room temperature" which is generally 20-22°C. However, ambient temperature of a "warm room" (for bacteriological growth) can be 37°C. Thus, ambient temperature is readily determined from the context in which it is used and is well understood by those of skill in the art.

*Formation of Delivery Systems*

The modified glycosides can be used to form a biodegradable delivery system, optionally with a substance incorporated therein, such as a therapeutic substance. The modified glycosides are referred to herein as the "vehicle" used to form the delivery system. As used herein, the term "delivery system" refers to any form of the modified glycoside having a substance incorporated therein. Preferably, the delivery system is in the form of an amorphous, glass matrix having the substance incorporated therein. The glass matrix advantageously can be designed to have a desired release rate of the substance incorporated therein, by selection of the material forming the matrix, selection of the conditions of forming the matrix, and by the addition of other substances which can modify the rate of release.

The modified glycosides readily form glasses either from a quenched melt or an evaporated organic solvent. Examples of methods of forming amorphous carbohydrate glass matrices are described in "Pharmaceutical Dosage Forms," Vol. 1 (H. Lieberman and L. Lachman, Eds.) 1982.

The modified glycosides in purified form and substance or substances to be incorporated can be intimately mixed together in the appropriate molar ratios and melted until clear. Suitable melting conditions include, but are not limited to, melting in open glass flasks between about 50 and 250 °C for about 1-2 minutes. This results in a fluid melt

which can be allowed to slightly cool before dissolving the substance in the melt, if required, and quenching to glass for instance by pouring over a brass plate or into a metal mould for shaped delivery vehicles. Melt temperature can be carefully controlled and substances can be incorporated into the modified glycosides either in the pre-melted formulation, or stirred into the cooling melt before quenching.

The melts are thermally stable and allow the incorporation of molecules without denaturation, or suspension of core particles without alteration of their physical nature. The glass melts can be used also to coat micron-sized particles, this is particularly important in the formulation of non-hygroscopic powders containing hygroscopic actives, for by-inhalation administration of therapeutic agents.

Alternatively, vitreous delivery systems can be formed by evaporation of the modified glycosides and substance to be incorporated in solution in a solvent or mixture of solvents. Suitable organic solvents include, but are not limited to, dichloromethane, chloroform, dimethylsulfoxide (DMSO), dimethylformamide (DMF) and higher alcohols. The exact nature of the solvent is immaterial as it is completely removed on formation of the delivery system. Preferably, both the modified glycoside and substance to be incorporated are soluble in the solvent. However, the solvent may dissolve the modified glycoside and allow a suspension of the substance to be incorporated in the matrix. Preferably, on concentrating the solvent, crystallization of the modified glycosides does not occur. Instead, an amorphous solid ("glass" or "glass matrix" herein) is produced, which has similar properties to the quenched glass. Substances can be incorporated easily either in solution or as a particle suspension.

A solution of the substance to be incorporated containing a sufficient quantity of modified glycoside to form a glass on drying can be dried by any method known in the art, including, but not limited to, freeze drying, vacuum, spray, belt, air or fluidized-bed drying. Another suitable method of drying, exposing a syrup to a vacuum under ambient temperature, is described in PCT GB96/01367. After formation of a glass containing homogeneously distributed substance in solid solution or fine suspension in the glass, the glasses can then be milled and/or micronized to give microparticles of homogeneous defined size.

Different dosing schemes can also be achieved by the delivery system formulated. The delivery system can permit a quick release or flooding dose of the incorporated substance after administration, upon the dissolving and release of the substance from the delivery system. Coformulations of vehicles with slowly water soluble glasses and plastics such as phosphate, nitrate or carboxylate glasses and lactide/glycolide, glucuronide or polyhydroxybutyrate plastics and polyesters, provide more slowly dissolving vehicles for a slower release and prolonged dosing effect. Optionally, a substance can be incorporated into the glass matrix which retards recrystallization of the matrix, such as polyvinylpyrrolidone, or a hydrophobic substance can be incorporated in the matrix, so as to modify the release rate of the substance, such as a water insoluble wax or a fatty acid WO 93/10758.

The delivery systems can also be coformulated with a hydrophobically-derivatized carbohydrate (HDC) glass forming material. HDC glass forming materials are described in PCT WO 96/03978. As used herein, HDC refers to a wide variety of hydrophobically derivatized carbohydrates where at least one hydroxyl group is substituted with a hydrophobic moiety. Examples of suitable HDCs and their syntheses are described in Developments in Food Carbohydrate - 2 ed. C.K. Lee, Applied Science Publishers, London (1980). Other syntheses are described for instance, in Akoh et al. (1987) J. Food Sci. 52:1570; Khan et al. (1993) Tetra. Letts 34:7767; Khan (1984) Pure & Appl. Chem. 56: 833-844; and Khan et al. (1990) Carb. Res. 198:275-283.

The delivery of more than one bioactive material can also be achieved using a delivery system including multiple coatings or layers loaded with different materials or mixtures thereof. Administration of the solid dose delivery systems of the present invention can be used in conjunction with other conventional therapies and coadministered with other therapeutic, prophylactic or diagnostic substances, coadministered with other therapeutic, prophylactic or diagnostic substances.

The solid delivery systems can be used to deliver therapeutic agents by any means including, but not limited to, topical, transdermal, transmucosal, oral, gastrointestinal, intraperitoneal, subcutaneous, ocular, intramuscular, intravenous and by-inhalation (naso-pharyngeal and pulmonary, including transbronchial and transalveolar).

Topical administration is, for instance, by a dressing or bandage having dispersed therein a delivery system, or by direct administration of a delivery system into incisions or open wounds. Creams or ointments having dispersed therein slow release bead or microspheres of a delivery system are suitable for use as topical ointments or wound filling agents.

Compositions for transdermal administration are preferably powders of delivery systems in the form of preferably homogeneously sized microneedles or microbeads. Larger, macroscopic needle and bead forms of the delivery systems are also provided for subdermal implantation and extended drug delivery. The particle sizes should be small enough so that they cause only minimal skin damage upon administration. The powders can be prepackaged in single-dose, sealed, sterile formats. Suitable methods of transdermal administration include, but are not limited to, direct impact, ballistic, trocar and liquid jet delivery.

The delivery systems suitable for transmucosal delivery include, but are not limited to, mucoadhesive wafers, films or powders, lozenges for oral delivery, pessaries, and rings and other devices for vaginal or cervical delivery.

Compositions suitable for gastrointestinal administration include, but are not limited to, pharmaceutically acceptable powders, tablets, capsules and pills for ingestion and suppositories for rectal administration.

Compositions suitable for subcutaneous administration include, but are not limited to, various implants. Preferably the implants are macroscopic discoid, release. Since the entire implant is dissolved in the body fluids, removal of the implant is not necessary. Furthermore, the implants do not contain synthetic polymers and are biodegradable.

Compositions suitable for ocular administration include, but are not limited to, microsphere and macrosphere formulations and saline drops, creams and ointments containing these and round-ended shaped rods which fit comfortably in the lower conjunctival fornix beneath the lower eyelid.

Compositions suitable for by-inhalation administration include, but are not limited to, powder forms of the delivery systems. There are a variety of devices suitable for use in by-inhalation delivery of powders. See, e.g., Lindberg (1993) Summary of Lecture at Management Forum 6-7 December 1993 "Creating the Future for Portable Inhalers," Additional devices suitable for use herein include, but are not limited to, those described in WO 94/13271, WO 94/08552, WO 93/09832 and United States Patent No. 5,239,993.

The delivery systems are preferably biodegradable and release substances incorporated therein over a desired time period, depending on the particular application, and the composition of the system. As used herein, the term "biodegradable" refers to the ability to degrade under the appropriate conditions of use, such as outdoors, or in the body, for example by dissolution, devitrification, hydrolysis or enzymatic reaction.

*Substances Incorporated in the Delivery Systems*

Substances which can be incorporated into the delivery systems include, but are not limited to, industrial chemicals such dyes and perfumes, as well as medicinal or agricultural bioactive materials suitable for use *in vivo* and *in vitro.* Suitable bioactive materials include, but are not limited to, pharmaceutical agents, therapeutic and prophylactic agents, and agrochemicals such as pesticides and pheromones, and prophylactic agents, and agrochemicals such as pesticides and pheromones.

Suitable therapeutic and prophylactic agents include, but are not limited to, any therapeutically effective biological modifier. Such modifiers include, but are not limited to, pharmaceutical actives, subcellular compositions, cells, bacteria, viruses and molecules including, but not limited to, lipids, organics, proteins and peptides (synthetic and natural), peptide mimetics, hormones (peptide, steroid and corticosteroid), D and L amino acid polymers, saccharides including oligosaccharides and polysaccharides, nucleotides, oligonucleotides and nucleic acids, including DNA and RNA, protein-nucleic acid hybrids, small molecules and physiologically active analogs thereof. Further, the modifiers can be derived from natural sources or made by recombinant or synthetic means and include analogs, agonists and homologs.

As used herein "protein" refers also to peptides and polypeptides. Such proteins include, but are not limited to, enzymes, biopharmaceuticals, growth hormones, growth factors, insulin, monoclonal antibodies, interferons, interleukins and cytokines.

Organic compounds include, but are not limited to, pharmaceutically active chemicals. For instance, representative organic compounds include, but are not limited to, vitamins, neurotransmitters, antimicrobials, antihistamines, analgesics and immunosuppressants.

Compositions comprising solid dose delivery systems containing prophylactic bioactive materials and carriers therefore are further encompassed by the invention. Preferable compositions include immunogens such as for use in vaccines. Preferably, the compositions contain an amount of the immunogen effective for either immunization or booster inoculation.

*Formation of Colored Optically Clear Devices and Coatings*

In one embodiment, the modified glycosides are mixed with an appropriate amount of an optically active dye, such as oil red, and the mixture melted. The dissolution of the active is accompanied by a sharp intensification of colour and the melt is then quenched to yield an optically clear coloured glass which can be used, for example, as a filter device or coating. For example, the melt containing the dissolved dye can be applied to a surface, such as a plastic surface of any of a variety of devices, by methods known in the art such as dip coating or spraying to yield a coating of optically clear coloured glass. In another embodiment, optically active material is added directly to the melt of the modified glycosides for dissolution prior to formation of the optically clear colored glass device or coating. Preferably, the optically clear devices and coatings are not biodegradable.

In yet another embodiment, the modified glycosides and the optically active material to be incorporated are dissolved in a compatible solvent, and the solvent is removed by evaporation to form the optically clear coloured glass. Alternatively, the solution containing both modified glycoside and active is applied directly to a material or device by dip coating or spraying to yield a coating of optically clear coloured glass on evaporation of the solvent. In general, the

tendency to crystallize, from the melt or with reduction of solvent content, is low ensuring the long-term integrity of the glass device or coating and the process of devitrification can be further controlled by modifiers such as other derivative sugars and certain organic compounds including hydrophobically derivatized compounds.

Many applications are encompassed by the intensified colored glass of the present invention. For example, in the plastics industry, enhanced coloring is desirable in the manufacture of a wide range of articles such as dishware, medical devices, films, and tubing. Additionally, the coloring of synthetic fibers could be enhanced for use in clothing and carpet manufacture. Methods to incorporate the intensified colored glass with a given plastic are discussed below.

*Extrusion and Injection Molding*

One method of coloring plastic is accomplished by mixing pre-colored plastic, with uncolored (virgin) plastic. The process is well known in the art and generally described in U.S. Patent No. 3,930,640 to Kuehn et al. For ease of weighing, storage, and shipping, the plastics are typically pelletized. More specifically, the pellets are formed as strands of plastic and are cut to a short length after being extruded through a circular die.

A variety of ways exist to mix the pre-colored pellets and the virgin pellets. Typically, the pellets are fed into a heated barrel of an extrusion or injection molding machine. Such barrels contain at least one longitudinal screw for moving and mixing the different pellets, forming a homogeneous melt. Once in a melted state, the plastic is forced through an extruding die or alternatively, into an injection mold cavity.

Referring to the present invention, the intensified colored glass could be advantageously used as a coloring agent in the colored pellets or perhaps, directly as the pre-colored pellets. Regardless of whether the colored glass is used as a coloring agent or the actual pre-colored pellets, the colored glass would have to be a convenient shape; e.g., small spheres, granules, cylinders, pellets, etc. which would facilitate ease of handling and eliminate problems associated with dust. Once the colored glass of the present invention was in a conveniently sized form, both the pre-colored and virgin pellets could be appropriately combined or "compounded" to achieve the desired color intensity. Normally, the pre-colored plastic pellets are loaded with high concentrations of dye or pigment. For this reason, people in the trade often refer to pre-colored plastic as "color concentrates." In summary, adequate coloring of the final product may be realized by combining the pre-colored and virgin pellets in a ratio as low as 1:50, but more preferably 1:5.

Central to the overall coloring process is the compounding stage. Of the approaches to compounding, dry blending is frequently employed. In dry blending, pigment or dye in a powdered form is added to the virgin pellets in a large drum or barrel. The drum is tumbled or shaken to blend the additives with the pellets. Electrostatic forces may also encourage the dyes to adhere to the pellets. Subsequently, the coated pellets are fed into an extruder and repelletized in color concentrated form.

Alternatively, the additives may be mixed with the pellets in a melted state. For instance, the Banbury mixer (the first of the class of internal intensive mixers) mixes the materials in a closed chamber with two rotors which keep the material in constant circulation. High shear forces are imposed on the materials which enhance dispersion of the coloring agents. Despite the favorable mixing, the internal intensive mixers discharge product in the form of inconsistent lumps.

Wax has also been used to optimize the blending process. In U.S. Patent No. 4,173,492 to Pollard, wax is melted and mixed with pigments creating a uniform color. Subsequently, the wax is cooled to form a large mass which then may be fragmented into flakes or granules. Virgin plastic pellets are then tumbled or blended with the bulkier colored wax particulate prior to being introduced into a hopper of the extruding or molding machine.

Dissolving plastics in an appropriate solvent is another method of compounding coloring agents and plastic. After dissolving the plastic, the coloring agent is added. As the liquid is mixed, the coloring agent is uniformly dispersed. Subsequently, the solvent is flashed off. The use of solvents for compounding, however, is not as simple or practical as using the standard method of adding color concentrates immediately prior to extrusion or molding.

In summary, where internal uniform coloring is necessary, the use of color concentrates is desirable. Examples where uniform coloring is most desirable include high wear products such as: synthetic clothing and carpeting fibers, dishware, toys, automobile parts, etc. Furthermore, weighing and cleanup procedures shall be improved since problems associated with dust are avoided. On the other hand, color concentrates often melt at a lower temperature than the virgin plastic, causing the coloring to slide down the heated barrel of an extruder or injection mouding machine, as a "slug". Slugs create inconsistencies and should be avoided.

It should be appreciated that the present invention can improve the present color concentrate technology. First, the ability to control the softening or melting temperature of the colored glass of the instant invention may be useful to better match the melting points of various high melting point plastics, thereby ensuring uniform mixing and avoiding the formation of color "slugs." In addition, the high intensity coloring effect permits lower costs, since less coloring agent would be required. Lastly, the present invention has been shown to have good color stability, preventing early clouding and other color stability problems.

*Blow Molding, Coextrusion, and Other Applications*

In certain other applications, uniform internal coloring is not required. For instance, plastic bottles and containers may have multiple layers. Typically, a plastic bottle is extrusion blow molded and often consists of more than one layer. Utilizing a coextrusion blow molding process, dull recycled plastic may be masked by covering with a higher quality colored virgin plastic layer. Though the coextrusion and blow molding processes have different fundamental steps than their less complex counterparts, coextrusion and blow molding both utilize color concentrates and hence, could benefit by the use of intensified colored glass of the present invention.

*Sputter Coating*

For an even higher degree of thin film control, the well known technique of sputter coating can be employed. A sputtering effect is caused by a coating material being bombarded by ions. As a result of ion impact, kinetic energy is transferred to the surface atoms and breaks the chemical bonds which hold the surface atoms to neighboring atoms. Once the chemical bonds are overcome, the surface atoms are ejected. By placing a substrate in the path of the ejected atoms, a highly controlled thin film can be deposited onto the substrate.

Sputter coating of complex materials can be accomplished. McGraw-Hill Encyclopedia of Science and Technology, 279 (6th ed. 1987). For example, an inorganic composite such as borosilicate glass is sputtered for use in the semiconductor industry. Berenschot (1994) Sensors and Actuators A-Physical 41:338-342.

In addition to the semiconductor industry, sputtering applications include, but are not limited to, windows, packaging materials, filters, and polymeric materials. With such diversity, it follows that numerous products can be improved using the enhanced colored glass of the present invention.

Additionally, sputtering process can be performed in continuous or batch applications. In conventional applications, large devices to be coated are moved along a path opposite of the bombarded target material. U.S. Patent No. 5,507,931 describes a procedure where the coating metal is melted and sputtered while still in a molten state. Just as metals are sputtered in continuous processes, so can the enhanced glass matrices of the present invention, thus, making the benefits of enhanced coloring available for large commercial applications.

The invention will be further understood by the following non-limiting examples.

**Example 1: Synthesis and characterization of derivatives of glycosides of sugar alcohols.**

Acetyl derivatives of polyols were prepared, wherein the polyols were the following glycosides of sugar alcohols: lactitol, palatinit, and the individual sugar components of palatinit, as described below

10 g of the polyol was dissolved in 40 mL of acetic anhydride containing 4 g of sodium acetate. When all the sugar had dissolved, 100 mL of distilled water was added to the solution and the mixture extracted with dichloromethane to extract the derivatized polyol. The acetylated polyol was recovered by evaporating off the solvent and the derivative characterized by nuclear magnetic resonance spectroscopy (NMR) and differential scanning calorimetry (DSC). The products were obtained and characterized by NMR and DSC, for the acetyl derivatives of lactitol (4-O-β-D-galactopyranosyl-D-glucitol), palatinit [a mixture of GPS (α-D-glucopyranosyl-1→6-sorbitol) and GPM (α-D-glucopyranosyl-1→6-mannitol)], and its individual sugar alcohol components GPS and GPM. Table 1 shows the melting points and Tgs (glass transition state temperatures) for the acetyl derivatives formed, lactitol nonaacetate, palatinit nonaacetate and GPS nonaacetate and GPM nonaacetate.

The glasses showed a range of melt temperatures suitable for the incorporation of labile substances such as biologically active compounds. without thermal degradation, and Tgs above ambient. Tgs were obtained as the average of two runs.

Table 1

| Derivative | Melting Point | Tg |
|---|---|---|
| Lactitol nonaacetate | 119°C | 39.5°C |
| Palatinit nonaacetate | 108°C | 35.3 °C |
| GPS nonaacetate | 204°C | 17.4°C |
| GPM nonaacetate | 87°C | 35°C |

**Example 2: Formation of Glasses using Derivatives of Glycosides of Sugar Alcohols and Analysis of their Solvent Properties.**

Glasses are formed of the derivatives of glycosides of sugar alcohols prepared as described above in Example 1 by quenching from the melt according to the method described in WO 96/03978. Various dyes are added to the melts and then mixed before quenching to form glasses incorporating the dyes. Solubility of the dyes in the melt and in the quenched glass is assessed visually as an increase in dye intensity and the presence of particulate material. The results of the incorporation of various dyes in lactitol nonaacetate glasses is shown in Table 2; these glasses were also characterized by DSC. The glasses were found to be good solvents for poorly water soluble solutes. Surprisingly, the incorporation of such active substances showed little effect on the Tg of the glasses formed as assessed by DSC and no evidence of devitrification was observed even after 2 months at ambient temperature and humidity. The glasses are thus suitable for use as optically clear filter devices and for the encapsulation and controlled release of bioactive substances.

Table 2

| Dye | Water Solubility | glass solubility |
|---|---|---|
| Napthol green B | ± | ± |
| Mordant blue 9 | + | - |
| Acid yellow 65 | ++ | -- |
| Disperse red 1 | -- | +++ |

**Example 3: Formation of Glassy coating containing optically active molecules dissolved in modified glycosides**

Disperse Red 1 and lactitol nonaacetate (ratio 0.1/100 g/g) were dissolved in dichloromethane and the solution was applied in a fine coat to a glass slide. The solvent was evaporated off in an air stream, leaving a thin coating of red coloured lactitol nonaacetate glass. The coloured glass formed was visually compared to glasses of trehalose and trehalose acetate encapsulating the dye. The glass colour was more intense than an equivalent trehalose glass containing 10X more dye. Furthermore, the glass coating remained clear when exposed to ambient temperatures and humidities unlike glasses of trehalose octacetate which became cloudy overnight. The thin glass coating (>0.1 mm) remained clear, with no evidence of devitrification, even after storage under ambient conditions of temperature (fluctuations between approx. -10°C to 45°C) and humidity (fluctuation between approx. 5-95% R.H.) for at least 1 year.

**Example 4: Formation of glassy matrix of modified glycosides containing pharmaceutically active molecules for controlled release.**

The hydrohobic drug Cyclosporin A and the hydrophilic drug Diltiazem HCL were both incorporated in a glass of lactitol nonaacetate (ratio of 10g drug to 100g modified glycoside) by either quenching from the melt or evaporation from solvent. For glass formation by quenching from the melt, 1g of drug was dissolved in 10g of lactitol nonaacetate melt at 120°C and the mix quenched immediately after it went clear. For glass formation by solvent evaporation, 1g of drug and 10g of lactitol nonaacetate were dissolved in dichloromethane and the solvent was evaporated off in an air stream. Surprisingly, the glasses formed by both methods, and incorporating both drugs, were all optically clear and remained clear on storage at ambient temperatures and humidities for at least a month. Also surprisingly the Tgs of the glasses were all approx. 39°C. However, the glasses containing the different drugs showed different profiles of controlled release of incorporated drugs on immersion of the glasses in stirred saline solution. Addition of 0.1% detergent (Tween 20) to the saline solution also altered the rate of controlled release of incorporated drug.

**Example 5: Controlled release of active molecules dissolved in modified glycosides**

Disperse Red 1 was incorporated as a model active in a glass of lactitol nonaacetate (ratio 0.1/100 g/g) by melt mixing. The release of model active from 0.5mm and 3mm thickness layers of glass, into either water or an aqueous solution of 5% tween 20, was monitored by absorbance at 510nm. No dissolution of either thickness glass was observed in water whereas complete release of active was observed in 12 and 72 hours, from the 0.5mm and 3mm thickness glasses respectively, in surfactant containing media with constant agitation.

Although the foregoing invention has been described in some detail by way of illustration and example for purposes of clarity and understanding, it will be apparent to those skilled in the art that certain changes and modifications

may be practiced. Therefore, the description and examples should not be construed as limiting the scope of the invention, which is delineated by the appended claims.

## Claims

1. A composition comprising a modified glycoside and a substance to be released from the composition, wherein the modified glycoside has the formula:

$$(Y)_n\text{-}X$$

wherein

Y represents a saccharide subunit, n is 1-6, and each of the n saccharide subunits are linked in a linear or branched chain by one or more glycosidic linkages; and
X is a 5 or 6 carbon monosaccharide polyalcohol,

wherein a hydroxy group of the polyalcohol is linked via a glycosidic bond to the anomeric carbon of one of the saccharide subunits; and
wherein at least a portion of the hydroxy groups of the glycoside are derivatised in the form of an ester, mixed ester, ether or mixed ether.

2. A composition according to claim 1, wherein the saccharide subunits, Y, are the same or different and are selected from glucose, galactose, fructose, ribulose, mannose, ribose, arabinose, xylose, lyxose, allose, altrose and gulose.

3. A composition according to claim 1 or claim 2, wherein the polyalcohol is selected from erythritol, ribitol, xylitol, galactitol, glucitol and mannitol.

4. A composition according to claim 1, wherein the modified glycoside is selected from lactitol nonaacetate, palatinit nonaacetate, glycopyranosyl sorbitol nonaacetate, glucopyranosyl mannitol nonaacetate, maltitol nonaacetate and mixtures thereof.

5. A composition according to any preceding claim, wherein the modified glycoside is in the form of a vitreous glass matrix having the substance incorporated therein.

6. A composition according to claim 5, further comprising at least one physiologically acceptable glass selected from carboxylate, nitrate, sulfate, bisulfate, a hydrophobic carbohydrate derivative, and combinations thereof.

7. A composition according to any preceding claim, in the form of a solid delivery system selected from lozenge, tablet, disc, film, suppository, needle, microneedle, microfiber, particle, microparticle, sphere, microsphere, powder, and an implantable device.

8. A composition according to any preceding claim, for therapeutic use, wherein the substance is a pharmaceutically active chemical.

9. A composition according to any preceding claim, wherein the substance is selected from lipids, proteins, peptides, peptide mimetics, hormones, saccharides, nucleic acids, and protein nucleic acid hybrids.

10. A composition according to claim 9, wherein the substance is a protein selected from enzymes, growth hormones, growth factors, insulin, monoclonal antibodies, interferons, interleukins and cytokines.

11. A composition according to claim 8, wherein the substance is immunogenic and is selected from live viruses, attenuated viruses, nucleotide vectors encoding antigens, bacteria, antigens, antigens plus adjuvants and haptens coupled to carriers.

12. An optically clear device comprising a modified glycoside as defined in any of claims 1 to 4.

**13.** A device according to claim 12, further comprising an optically active, poorly water-soluble dye.

**14.** A surface-coated product, wherein the surface comprises plastics or metal, and the coating comprises a modified glycoside as defined in any of claims I to 4.

**15.** A product according to claim 14, further comprising an optically active, poorly water-soluble dye.

**16.** A method of making a vitreous solid delivery system having incorporated therein a substance to be released therefrom, the method comprising processing a modified glycoside as defined in any of claims 1 to 4, which is capable of forming a vitreous glass, with the substance.

**17.** A method according to claim 16, wherein the processing comprises melting the modified glycoside and incorporating the substance in the melt, wherein the melt temperature is sufficient to fluidise the modified glycoside, and insufficient to substantially inactivate the substance, and then quenching the melt.

**18.** A method according to claim 16, wherein the processing comprises dissolving or suspending the modified glycoside and the substance in a solvent effective in dissolving at least one of the modifi8ed glycoside and the substance, and evaporating the solvent.

**19.** A method according to any of claims 16 to 18, wherein the processing further comprises incorporating into the glass matrix at least one physiologically acceptable glass as defined in claim 6.

**20.** A method according to any of claims 16 to 19, wherein system is in a form defined in claim 7.

**21.** A method according to any of claims 16 to 20, wherein the substance is as'defined in claim 8 or claim 9.

**22.** A method of forming an optically clear glass having an optically active, poorly water-soluble dye incorporated therein, comprising processing the dye with a modified glycoside as defined in any of claims 1 to 4.

**23.** A method according to claim 22, wherein the glass comprises a filter device.

**24.** A method according to claim 22 or claim 23, which further comprises forming a coating of the optically clear glass on a surface.

**25.** A method according to claim 24, wherein the surface is of plastics or metal.

**Patentansprüche**

**1.** Zusammensetzung, umfassend ein modifiziertes Glycosid und eine aus der Zusammensetzung freizusetzende Substanz, wobei das modifizierte Glycosid die Formel

$$(Y)_n\text{-}X$$

aufweist, in der

Y eine Saccharid-Untereinheit darstellt, n für 1 - 6 steht und alle n Saccharid-Untereinheiten in einer linearen oder verzweigten Kette durch eine oder mehrere glycosidische Verknüpfungen verknüpft sind; und
X ein Monosaccharidpolyalkohol mit 5 oder 6 Kohlenstoffen ist,

wobei eine Hydroxygruppe des Polyalkohols über eine glycosidische Bindung mit dem anomeren Kohlenstoff einer der Saccharid-Untereinheiten verknüpft ist und
wobei mindestens ein Teil der Hydroxygruppen des Glycosids in Form eines Esters, gemischten Esters, Ethers oder gemischten Ethers derivatisiert ist.

**2.** Zusammensetzung nach Anspruch 1, in der die Saccharid-Untereinheiten Y gleich oder verschieden sind und aus Glucose, Galactose, Fructose, Ribulose, Mannose, Ribose, Arabinose, Xylose, Lyxose, Allose, Altrose und Gulose

ausgewählt sind.

3. Zusammensetzung nach Anspruch 1 oder Anspruch 2, in der der Polyalkohol aus Erythrit, Ribit, Xylit, Galactit, Glucit und Mannit ausgewählt ist.

4. Zusammensetzung nach Anspruch 1, in der das modifizierte Glycosid aus Lactitnonaacetat, Palatinitnonaacetat, Glycopyranosylsorbitnonaacetat, Glucopyranosylmannitnonaacetat, Maltitnonaacetat und deren Mischungen ausgewählt ist.

5. Zusammensetzung nach irgendeinem der vorangehenden Ansprüche, in der das modifizierte Glycosid in Form einer glasartigen Matrix vorliegt, welcher die Substanz einverleibt ist.

6. Zusammensetzung nach Anspruch 5, weiter umfassend mindestens ein physiologisch annehmbares Glas, das aus Carboxylat, Nitrat, Sulfat, Bisulfat, einem hydrophoben Kohlehydrat-Derivat und deren Kombinationen ausgewählt ist.

7. Zusammensetzung nach irgendeinem vorangehenden Anspruch in Form eines festen Zufuhrsystems, das aus Pastille, Tablette, Scheibe, Film, Suppositorium, Nadel, Mikronadel, Mikrofaser, Teilchen, Mikroteilchen, Kugel, Mikrokugel, Pulver und einer implantierbaren Vorrichtung ausgewählt ist.

8. Zusammensetzung nach irgendeinem vorangehenden Anspruch zur therapeutischen Verwendung, in der die Substanz eine pharmazeutisch aktive Chemikalie ist.

9. Zusammensetzung nach irgendeinem vorangehenden Anspruch, in der die Substanz aus Lipiden, Proteinen, Peptiden, Peptid-Mimetika, Hormonen, Sacchariden, Nucleinsäuren und Protein-Nucleinsäure-Hybriden ausgewählt ist.

10. Zusammensetzung nach Anspruch 9, in der die Substanz ein Protein ist, das aus Enzymen, Wachstumshormonen, Wachstumsfaktoren, Insulin, monoklonalen Antikörpern, Interferonen, Interleukinen und Cytokinen ausgewählt ist.

11. Zusammensetzung nach Anspruch 8, in der die Substanz immunogen ist und aus lebenden Viren, abgeschwächten Viren, Nucleotid-Vektoren, die für Antigene codieren, Bakterien, Antigenen, Antigenen plus Adjuvantien und an Träger gekoppelten Haptenen ausgewählt ist.

12. Optisch klare Vorrichtung, umfassend ein modifiziertes Glycosid, wie in irgendeinem der Ansprüche 1 bis 4 definiert.

13. Vorrichtung nach Anspruch 12, weiter umfassend einen optisch aktiven, schwer in Wasser löslichen Farbstoff.

14. Oberflächen-beschichtetes Produkt, in dem die Oberfläche Kunststoff oder Metall umfasst und die Beschichtung ein modifiziertes Glycosid, wie in irgendeinem der Ansprüche 1 bis 4 definiert, umfasst.

15. Produkt nach Anspruch 14, weiter umfassend einen optisch aktiven, in Wasser schlecht löslichen Farbstoff.

16. Verfahren zur Herstellung eines glasartigen festen Zufuhrsystems, dem eine daraus freizusetzende Substanz einverleibt ist, wobei das Verfahren umfasst, dass man ein modifiziertes Glycosid, wie in irgendeinem der Ansprüche 1 bis 4 definiert, das ein glasartiges Glas bilden kann, mit der Substanz verarbeitet.

17. Verfahren nach Anspruch 16, in dem die Verarbeitung das Schmelzen des modifizierten Glycosids und die Einverleibung der Substanz in die Schmelze, wobei die Schmelztemperatur ausreichend ist, um das modifizierte Glycosid zu fluidisieren, und nicht ausreichend ist, um die Substanz wesentlich zu desaktivieren, und das anschließende Quenchen der Schmelze umfasst.

18. Verfahren nach Anspruch 16, in dem die Verarbeitung das Lösen oder Suspendieren des modifizierten Glycosids und der Substanz in einem Lösungsmittel, das wirksam ist, mindestens ein Mitglied aus dem modifizierten Glycosid und der Substanz zu lösen, und das Verdampfen des Lösungsmittels umfasst.

19. Verfahren nach irgendeinem der Ansprüche 16 bis 18, in dem die Verarbeitung weiter umfasst, dass man der

Glasmatrix mindestens ein physiologisch annehmbares Glas, wie in Anspruch 6 definiert, einverleibt.

**20.** Verfahren nach irgendeinem der Ansprüche 16 bis 19, in dem das System in einer in Anspruch 7 definierten Form vorliegt.

**21.** Verfahren nach irgendeinem der Ansprüche 16 bis 20, in dem die Substanz wie in Anspruch 8 oder Anspruch 9 definiert ist.

**22.** Verfahren zur Bildung eines optisch klaren Glases, dem ein optisch aktiver, schlecht in Wasser löslicher Farbstoff einverleibt ist, umfassend das Verarbeiten des Farbstoffs mit einem modifizierten Glycosid, wie in irgendeinem der Ansprüche 1 bis 4 definiert.

**23.** Verfahren nach Anspruch 22, in dem das Glas ein Filtermittel umfasst.

**24.** Verfahren nach Anspruch 22 oder Anspruch 23, welches weiter das Bilden einer Beschichtung aus dem optisch klaren Glas auf einer Oberfläche umfasst.

**25.** Verfahren nach Anspruch 24, in dem die Oberfläche aus Kunststoff oder Metall ist.

## Revendications

**1.** Composition comprenant un glycoside modifié et une substance qui doit être libérée de la composition, dans laquelle le glycoside modifié a la formule:

$$(Y)_n\text{-}X$$

dans laquelle

Y représente une sous-unité saccharide, n vaut de 1 à 6, et les n sous-unités saccharide sont chacune liées en une chaîne linéaire ou ramifiée par une ou plusieurs liaisons glycoside; et
X est un polyalcool monosaccharide comptant de 5 à 6 atomes de carbone,

dans laquelle un groupe hydroxyle du polyalcool est lié par une liaison glycoside au carbone anomérique de l'une des sous-unités saccharide; et

dans laquelle au moins une partie des groupes hydroxyle du glycoside est dérivatisée sous la forme d'un ester, d'un ester mixte, d'un éther ou d'un éther mixte.

**2.** Composition selon la revendication 1, dans laquelle les sous-unités saccharide Y sont identiques ou différentes, et sont choisies parmi le glucose, le galactose, le fructose, le ribulose, le mannose, le ribose, l'arabinose, le xylose, le lyxose, l'allose, l'altrose et le gulose.

**3.** Composition selon la revendication 1 ou la revendication 2, dans laquelle le polyalcool est choisi parmi l'érythritol, le ribitol, le xylitol, le galacticol, le glucitol et le mannitol.

**4.** Composition selon la revendication 1, dans laquelle le glycoside modifié est choisi parmi le nonaacétate de lacticol, le nonaacétate de palatinite, le nonaacétate de glycopyranosyl sorbitol, le nonaacétate de glycopyranosyl mannitol, le nonaacétate de maltitol et des mélanges de ceux-ci.

**5.** Composition selon l'une quelconque des revendications précédentes, dans laquelle le glycoside modifié présente la forme d'une matrice de verre vitreux
dans laquelle est incorporée la substance.

**6.** Composition selon la revendication 5, comprenant en outre au moins un verre physiologiquement acceptable choisi parmi un dérivé de carboxylate, de nitrate, de sulfate, de bisulfate, d'un hydrate de carbone hydrophobe, et des combinaisons de ceux-ci.

**7.** Composition selon l'une quelconque des revendications précédentes, sous la forme d'un système d'administration solide choisi parmi une pastille, un comprimé, un disque, un film, un suppositoire, une aiguille, une micro-aiguille, une micro-fibre, une particule, une micro-particule, une sphère, une microsphère, une poudre et un dispositif implantable.

**8.** Composition selon l'une quelconque des revendications précédentes, à usage thérapeutique, dans laquelle la substance est un produit chimique pharmaceutiquement actif.

**9.** Composition selon l'une quelconque des revendications précédentes, dans laquelle la substance est choisie parmi des lipides, des protéines, des peptides, des mimétiques de peptide, des hormones, des saccharides, des acides nucléiques et des hybrides de protéine et d'acide nucléique.

**10.** Composition selon la revendication 9, dans laquelle la substance est une protéine choisie parmi des enzymes, des hormones de croissance, des facteurs de croissance, l'insuline, des anticorps monoclonaux, des interférons, des interleukines et des cytokines.

**11.** Composition selon la revendication 8, dans laquelle la substance est immunogène et est choisie parmi des virus vivants, des virus atténués, des vecteurs de nucléotides codant des antigènes, des bactéries, des antigènes, des antigènes additionnés d'adjuvants et des haptènes couplés à des porteurs.

**12.** Dispositif optiquement transparent comprenant un glycoside modifié selon l'une quelconque des revendications 1 à 4.

**13.** Dispositif selon la revendication 12, comprenant en outre un colorant optiquement actif, peu soluble dans l'eau.

**14.** Produit revêtu en surface, dans lequel la surface comprend un plastique ou un métal, et le revêtement comprend un glycoside modifié selon l'une quelconque des revendications 1 à 4.

**15.** Produit selon la revendication 14, comprenant en outre un colorant optiquement actif, peu soluble dans l'eau.

**16.** Procédé de fabrication d'un système d'administration solide vitreux, dans lequel est incorporée une substance qui doit en être libérée, le procédé comprenant le traitement d'un glycoside modifié selon l'une quelconque des revendications 1 à 4, qui est capable de former un verre vitreux avec la substance.

**17.** Procédé selon la revendication 16, dans lequel le traitement comprend les étapes consistant à faire fondre le glycoside modifié, et à incorporer la substance dans le produit fondu, la température du produit fondu étant suffisante pour fluidifier le glycoside modifié, et n'étant pas suffisante pour inactiver sensiblement la substance, et ensuite à refroidir rapidement le produit fondu.

**18.** Procédé selon la revendication 16, dans lequel le traitement comprend l'étape consistant à dissoudre ou à mettre en suspension le glycoside modifié et la substance, dans un solvant efficace pour dissoudre au moins l'un parmi le glycoside modifié et la substance, et à évaporer le solvant.

**19.** Procédé selon l'une quelconque des revendications 16 à 18, dans lequel le traitement comprend en outre l'étape consistant à incorporer dans la matrice de verre, au moins un verre physiologiquement acceptable tel que défini dans la revendication 6.

**20.** Procédé selon l'une quelconque des revendications 16 à 19, dans lequel le système se présente sous une forme définie dans la revendication 7.

**21.** Procédé selon l'une quelconque des revendications 16 à 20, dans lequel la substance est telle que définie dans la revendication 8 ou la revendication 9.

**22.** Procédé de formation d'un verre optiquement transparent dans lequel est incorporé un colorant optiquement actif, peu soluble dans l'eau, qui comprend l'étape consistant à traiter le colorant avec un glycoside modifié tel que défini par l'une quelconque des revendications 1 à 4.

**23.** Procédé selon la revendication 22, dans lequel le verre comprend un dispositif filtrant.

**24.** Procédé selon la revendication 22 ou la revendication 23, qui comprend en outre l'étape consistant à former un revêtement du verre optiquement transparent sur une surface.

**25.** Procédé selon la revendication 24, dans lequel la surface est en plastique ou en métal.